# EUROPEAN PATENT SPECIFICATION

(11) **EP 0 593 552 B1**
(45) Date of publication and mention of the grant of the patent: **03.09.1997**
(21) Application number: 92913973.1
(22) Date of filing: 30.06.1992
(51) Int. Cl.: B01J 2/14, B01J 2/16

(54) **PROCESS FOR PREPARING DRUG SUBSTANCES IN BEADLET FORM**
HERSTELLUNGSVERFAHREN VON MEDIKAMENTÖSE SUBSTANZEN IN GRANULATFORM
PROCEDE DE PREPARATION DE SUBSTANCES MEDICAMENTEUSES SOUS FORME DE GRANULES

(30) Priority: 03.07.1991 GB 9114374
(43) Date of publication of application: 27.04.1994
(73) Proprietor: SMITHKLINE BEECHAM PLC, Brentford, Middlesex TW8 9EP (GB)
(72) Inventor: HUGHES, John K., SmithKline Beecham Health Care, Merseyside, Lancashire WA10 1QL (GB); McNAIR, Mairi, SmithKline Beecham Consumer Brands, Weybridge, Surrey KT13 0DE (GB)
(74) Representative: White, Susan Mary
(86) International application number: GB9201185
(87) International publication number: WO9300991

(56) References cited:
- EP-A- 0 228 633
- EP-A- 0 361 874
- WO-A-88/01904
- US-A- 3 408 746

## Description

The present invention relates to a process for the manufacture of drug substances in the form of substantially spherical pellets (beadlets) having a high drug content and to products obtainable thereby.

The many advantages associated with the manufacture of drug substances in the form of solid, substantially spherical pellets or beadlets include, inter alia, ease of handling, the provision of a convenient substrate for subsequent coating in order, for example, to achieve taste-masking and/or sustained release properties, and the ability to combine two or more actives in a single dosage presentation. When ingested into the body, the additional potential benefits of pelleted products include maximisation of drug absorption and the avoidance of dose dumping.

Known methods for the manufacture of drugs in pelletised form include extrusion spheronisation from wet granulations and pan-coating techniques. For applications requiring a highly reproducible, free-flowing, high density, high potency, substantially spherical product, such methods have proved inadequate.

Uniform, substantially spherical particles can be obtained by the layering of drug from solution onto non-pareil seeds or granules using a centrifugal, fluidised bed, coater-granulator, known as a rotogranulator. Such apparatus comprises a rotation apparatus consisting of a variable speed; rotating disc (rotor) within a stationary cylinder (stator), with a slit between the stator and the rotor through which a fluidising air current is forced, e.g.: as in EP-A-0 228 633.

It has been found that beadlets prepared by this method do not meet the bulk density requirements of certain drug presentations. For example, in capsule dosage forms, the available volume has been found insufficient to accommodate the desired drug load. This problem is exacerbated when, for example, beadlets to be contained in compact capsules or formulated as tablets are prepared from drug substances having an inherently low density, when a significant proportion of binder is present in the beadlets, and for combination drug presentations in beadlet form.

The non-steroidal anti-inflammatory agent (NSAID) ibuprofen is typical of a compound for which formulation difficulties have been encountered. It is available as a crystalline powder (mp 75-77°C) which is only slightly soluble in water but readily soluble in solvents such as acetone and ethanol, and is inherently a low density material. Beadlets formed using standard rotating disc (spheroniser)/fluidised bed tray-dried techniques were found unsatisfactory; the bulk density of beadlets thus prepared was insufficient to achieve a target capsule (size 0) fill-weight in excess of 400mg. Moreover, the relatively low melting point of ibuprofen imposes limitations on the temperature of the air current for the fluidised bed and for drying the beadlets, once formed. The initially-formed beadlets incorporate a significant amount of solvent such that an extended post-preparation drying phase is required to ensure complete solvent removal.

The problem which the present invention sets out to solve is the provision of drug substances in beadlet form which have an increased bulk density when compared with beadlets made available by existing rotating disc (spheroniser)/fluidised bed techniques. This problem is solved according to the present invention which furthermore confers additional advantages with respect to overall processing time.

Accordingly, the present invention provides a process for the preparation of drug substances in beadlet form comprising:
forming a fluidised bed of particles of inert core material within the processing chamber of a coater-granulator apparatus by passing a gaseous current between the rotating disc and the inner wall of the processing chamber; and forming beadlets by layering a drug onto the particles of the core material by spray-coating with a solution of the drug; characterised in that during beadlet formation, the spray-coated particles are (i) transferred to a drying zone surrounding the processing chamber, (ii) carried through the drying zone by a gaseous current, and (iii) returned to the processing chamber; the recirculation process being continued until beadlets having the desired drug load are formed.

Coater-granulator apparatus suitable for carrying out the process of the present invention are commercially available or may be constructed by modification of standard fluidised bed granulators. In a preferred apparatus, an annular ring gap in the wall of the processing chamber is provided through which spray-coated particles are transferred to the drying zone. The physical characteristics of the rotor and stator are suitably designed to achieve optimum granulation and coating of beadlets, and a free-flowing, substantially spherical product in accordance with apparatus known in the art. A tangentially aligned, adjustable spray-rate, spray-coating system is preferred. A gaseous current required to form a particulate fluidised bed and transfer spray - coated beadlets within the coater-granulator during beadlet formation may comprise any suitable drying gas and is preferably an air current. The variable operating parameters for the coater-granulator apparatus are selected to optimise beadlet formation for a chosen drug substance. Key, variable operating parameters include rotor speed, atomising gas pressure, annular gap gas pressure, air volume, solution spray-rate and spray-gun nozzle diameter, slit width for the fluidised bed and inlet and outlet air temperatures.

An advantageous feature of the process of the invention is that an extended post-preparation drying phase is not required. By a suitable choice of operating temperatures, solvent evaporation takes place as beadlets are formed, during transport of the newly-drug-coated beadlets through the drying zone surrounding the processing chamber and during the subsequent recirculation. The process of the invention thus gives rise to a non-porous beadlet in which drug is bound to the core particle in uniform, dense layers. Interstices created in drug layers, when solvent evaporates during post-preparation drying according to known coater-granulator techniques, are not present in beadlets of the present invention. The beadlets obtainable by the process of the invention are structurally novel and accordingly form part of the invention.

The process of the present invention is amenable to the preparation of beadlets from both hydrophilic and lipophilic drug substances. Drugs particularly suited to processing in beadlet form according to the invention include ibuprofen, pseudoephedrine, phenylpropanolamine, chlorpheniramine, dextromethorphan and diphenhydramine including pharmaceutically acceptable salts thereof, for example ibuprofen, pseudoephedrine HCl, phenylpropanolamine HCl, chlorpheniramine maleate, dextromethorphan HBr and diphenhydramine HCl.

Solutions of drug substances for spray-coating onto inert core particles may be prepared from one or more solvents. Suitable solvents or solvent mixtures are those which both dissolve the selected drug substance and which are sufficiently volatile to evaporate at the operating temperature of the coater-granulator apparatus. Preferred solvents include acetone, water or aliphatic alcohols, for example lower alkyl aliphatic alcohols such as ethanol, or mixtures thereof.

Beadlets prepared according to the invention may comprise a single drug substance or mixtures of two or more substances. Different drugs may be layered sequentially onto core particles, or alternatively, where a single solvent system is compatible with two or more drugs, drug combinations may be deposited in a single layer.

The concentration of drug in the spray-coating solution will be selected according to known coating procedures and will of course be determined to some extent by drug solubility in the chosen solvent system. Good results are generally obtained using a drug concentration in the range 20-60% w/w, preferably 25-45% w/w. For example, ibuprofen beadlets are suitably prepared from ethanol solution, preferably at a concentration of approximately 40% w/w. Water-soluble materials such as phenylpropanolamine HCl and pseudoephedrine HCl are conveniently formed into beadlets from aqueous solution at concentrations in the range 25-40% w/w.

Conventional binder materials, routinely used in wet-granulation techniques, may be incorporated into beadlets by mixing with drug in the coating solution. Suitable binder materials include gelatin, starch, polyvinylpyrrolidone (PVP), hydroxypropylcellulose (HPC), hydroxypropylmethylcellulose (HPMC) and sodium carboxymethylcellulose. Since it is a primary object of the present invention to maximise beadlet drug load, the amount of binder incorporated is preferably small, for example no more than 1.0% w/w and preferably less than 1.0% w/w of the coating solution.

Preferred core materials for use in the invention include commercially available non-pareil seeds with mesh size in the range 20-40#(850-410µm), for example in the ranges 20-25#(850-710µm), 25-30#(710-600µm), 30-35#(600-500µm), and 35-40#(500-410µm).

The size and bulk density of beadlets formed by coater-granulator techniques is influenced by the diameter of the core particles. Smaller core particles, for example 500-410µm(35-40#) non-pareils, produce a more compact beadlet than larger core particles, for example 850-710µm(20-25#) non-pareils. It has been shown by scanning electron photomicroscopy that beadlets formed from larger core particles have a more porous structure than beadlets formed from small particles. Bulk densities required for high drug loads in accordance with the present invention are therefore promoted by use of smaller core particles. (I. Ghebre-Sellassie et al.; Drug Devel.& Ind. Pharmacy, 11(8), 1523-1541, 1985.)

Experiments with certain drugs, for example ibuprofen, have however shown that there is a tendency for beadlets formed layering of drug onto small diameter non-pareils (eg 35-40# )(500-410µm) to cling together (clump) and float during dissolution testing.

It is a particular feature of the present invention that beadlets can be prepared from small diameter non-pareil seeds without the disadvantages of clumping and floating. This is achieved by reducing the quantity of binder material below the generally accepted level and preferably excluding it altogether.

Beadlets formed in the absence of binder material thus constitute part of the present invention.

Beadlets having a theoretical drug load in excess of 85%w/w and an actual fill weight in excess of 450mg, for example 480 to 490mg per size 0 capsule are obtainable according to the invention. Good quality, substantially spherical, free flowing beadlets having a smooth surface are produced when the spray-coating rate is carefully controlled, the best results being observed when spray-rate is increased throughout the coating process. Short drying times for example from 1 to 10 minutes after spray-coating is terminated are all that is generally required.

In a further aspect the present invention extends to the film-coating of beadlets formed by the process hereinbefore described, for example to provide a protective coating for taste-masking purposes or to provide controlled and/or sustained release of the active drug substance after dosing.

Coating operations may be performed using any suitable coating equipment known in the art. Coating may be carried out as a separate operation to that of preparing the drug beadlets *per se* using any suitable equipment. Alternatively beadlet preparation and coating may be carried out in a single operation, the coating step forming an extension of the beadlet forming process, using equipment of the type hereinbefore described.

Suitable film-coating materials include natural and synthetic polymer materials and polymer mixtures such as cellulose and acrylate based polymers, including commercially available materials. Examples of commercially available coating materials include those made available under the trade names Eudragit and Aquacoat. Coating is suitably carried out by spray-coating from a polymer emulsion or colloidal dispersion, for example from an aqueous colloidal dispersion of a polymer material.

The following Examples illustrate the process of the invention and beadlets obtainable thereby. The coater-granulator apparatus used for the preparation of beadlets as described in the Examples is an Aeromatic Roto-Processor (Multi-Processor MPI) made available by Niro Atomiser. The Roto-Processor was operated using the spinning disk, fluidised bed and drying zone transference functions in combination. Throughout all processing operations spray-gun diameter was set at 0.8mm, atomisation air pressure at 2.0 bar, and annular air gap pressure at 0.5 bar.

### Example 1

### Preparation of Ibuprofen Beadlets Using 20-25#(850-710µm) Non-Pareil Seeds

- Rotating Disc Speed: : 200 rpm
- Air Volume: : 5.0
- Inlet Temperature: : 60°C
- Outlet Temperature: : 40°C (dropping to 38°C)

2kg of non-pareils (20-25#)(850-710µm) were placed in the bowl of the rotoprocessor. A 40% solution of ibuprofen in industrial methylated spirit (IMS), containing 0.8% polyvinylpyrrolidone (PVP) was sprayed onto the non-pareils at a rate of 104g/min..

Ibuprofen beadlets with a fill weight of 508mg per size 0 capsule were obtained. 89.6% by weight of beadlets were shown by sieve analysis to have a diameter in the range 1000-1680µm.

### Example 2

### Preparation of Ibuprofen Beadlets Using 500-410µm(35-40#) Non-Pareil Seeds

1.5kg of 500-410µm(35-40#) non-pareils were placed in the bowl of the rotoprocessor. Ibuprofen solution as described in Example 1 was sprayed onto the non-pareils in three separate coating operations. The spray-rate was increased during each coating operation.

| | | |
|---|---|---|
| 1st Coat | Rotating disc speed | 200rpm |
| | Air vol. | 5.0 |

| Amount of solution sprayed on in g. | Inlet air temp.°C | Outlet air temp.°C | Spray rate g/min |
|---|---|---|---|
| 0 | 50 | 43 | 40 |
| 700 | 50 | 43 | 44 |
| 1000 | 50 | 43 | 51 |
| 1300 | 50 | 42 | 55 |
| 1800 | 55 | 43 | 55 |
| 2100 | 55 | 44 | 60 |
| 2400 | 55 | 44 | 62 |
| 2700 | 55 | 43 | 68 |
| 3800 | 55 | 42 | 68 |
| 5900 | 55 | 41 | 68 |

Spraying was stopped when the theoretical drug load had reached 62.5% w/w (6.25kg of solution applied). A drying time of 5 minutes then followed, with the outlet temperature being raised to 47°C. Sieve analysis showed that 99.5% of beadlets had a diameter in the range 500-1000um.
Yield of product = 4.062kg
Fill weight per size 0 capsule = 503mg

| | | |
|---|---|---|
| 2nd Coat | Rotating disc speed | 433rpm |
| | Inlet air temp. | 55° C |
| | Air vol. | 5.0-7.5 |

1.5kg of beadlets obtained from the 1st coating operation were coated with a further 6.25kg of ibuprofen solution.

| Amount of solution sprayed on in g. | Outlet air temp.°C | Spray rate g/min |
|---|---|---|
| 0 | 45 | 32 |
| 200 | 46 | 47 |
| 400 | 45 | 58 |
| 1000 | 44 | 65 |
| 2000 | 43 | 65 |
| 4000 | 43 | 65 |
| 6000 | 42 | 65 |
| 6100 | 41 | 65 |
| 6200 | 41 | 65 |

The outlet temperature was raised to 49°C during a drying time of 5mins. 94% by weight of the beadlets obtained had a diameter in the range 1000-2000um.
- Theoretical drug load: = 85.9%
- Yield of product: = 4.052kg
- Fill weight per size 0 capsule: = 449mg

| | | |
|---|---|---|
| 3rd Coat | Rotating disc speed | 433rpm |
| | Inlet air temp. | 55° C |
| | Air vol. | 5.0-8.0 |

1.5kg of ibuprofen beadlets obtained from the 2nd coating operation were coated with a further 6.25kg of ibuprofen solution.

| Amount of solution sprayed on in g. | Outlet air temp.°C | Spray temp. g/min |
|---|---|---|
| 0 | 43 | 32 |
| 300 | 45 | 54 |
| 600 | 46 | 69 |
| 1000 | 45 | 73 |
| 2800 | 45 | 73 |
| 3500 | 45 | 73 |
| 3800 | 45 | 73 |
| 4000 | 45 | 73 |
| 4050 | 45 | 73 |
| 4200 | 48 | 73 |
| 5000 | 46 | 73 |
| 6000 | 46 | 73 |

Beadlets were dried for 3 mins., whilst raising the outlet temperature to 49°C. 99.0% by weight of beadlets had a diameter in the range 1000-2000um.
- Theoretical drug load: = 94.7%
- Yield of product: = 4.038kg
- Fill weight per size 0 capsule: = 403mg

### Example 3

### Preparation of Ibuprofen Beadlets Using 500-410µm(35-40#) Non-Pareil Seeds in the Absence of Binder Material.

The process of Example 2 was repeated using a 40% w/w solution of ibuprofen in IMS but omitting the binder material. Three coating operations were carried out, each separate run using 6.25kg of ibuprofen solution. In the first run, a 1st ibuprofen layer was applied to the non-pareils. In the 2nd and 3rd runs, a second ibuprofen layer was applied to batches of the single-coat beadlets, under different operating conditions.

| | | |
|---|---|---|
| 1st Coat: | Rotating disc speed | 433rpm |
| | Air vol. | 4.0-6.5 |

| Time in mins. | Inlet air temp.°C | Outlet air temp.°C | Spray Rate g/min |
|---|---|---|---|
| 0 | 55 | 43 | 32 |
| 6 | 55 | 41 | 40 |
| 12 | 55 | 41 | 50 |
| 19 | 55 | 39 | 61 |
| 23 | 60 | 38 | 61 |
| 37 | 60 | 38 | 66 |
| 74 | 60 | 40 | 66 |
| 103 | 60 | 41 | 66 |

- Drying Time: = 5mins.
- Theoretical drug load: = 62.5%

| | | |
|---|---|---|
| 2nd Coat (a) | Rotating disc speed | 433rpm |
| | Air vol. | 4.0-10.5 |

| Time in mins. | Inlet air temp.°C | Outlet air temp.°C | Spray Rate g/min |
|---|---|---|---|
| 0 | 60 | 44 | 39 |
| 5 | 60 | 44 | 48 |
| 9 | 60 | 43 | 58 |
| 13 | 60 | 43 | 66 |
| 33 | 60 | 40 | 66 |
| 87 | 60 | 33 | 68 |
| 115 | 60 | 34 | 68 |
| 125 | 60 | 32 | 68 |
| 130 | 60 | 30 | 68 |
| 150 | 60 | 32 | 68 |

Spraying was complete after 150mins. Spraying was interupted after 20mins (10 mins) and after 34 mins. (45 mins).Beadlets were dried for 5 mins.
- Theoretical drug load: = 85.9% w/w
- Actual drug load: = 84.1%w/w
- Fill weight per size 0 capsule: = 487mg

| | | |
|---|---|---|
| 2nd Coat (b) | Rotating disc speed | 433rpm |
| | Air vol. | 3.0-4.7 |

| Time in mins. | Inlet air temp.°C | Outlet air temp.°C | Spray Rate g/min |
|---|---|---|---|
| 0 | 60 | 40 | 57 |
| 5 | 60 | 37 | 68 |
| 9 | 60 | 34 | 72 |
| 15 | 65 | 30 | 72 |
| 52 | 65 | 31 | 73 |
| 60 | 65 | 32 | 70 |
| 156 | 65 | 32 | 56 |
| 166 | 65 | 37 | 57 |
| 176 | 65 | 38 | 73 |

Beadlets were dried for 5 mins.
- Theoretical drug load: = 85.9% w/w
- Actual drug load: = 85.4% w/w
- Fill weight per size 0 capsule: = 485mg

96.06% by weight of beadlets were shown by sieve analysis to have a diameter in the range 710-1000µm.

### Example 4

### Preparation of Phenylpropanolamine Beadlets using 850-710µm(20-25#) Non-Pareil Seeds

1.5kg of non-pareil seeds 850-710µm(20-25#) were placed in the bowl of the rotoprocessor. 8.33kg of PPA HCl solution (27% w/w in filtered tap water) with no binder present was sprayed onto the seeds. The spray rate was increased during processing.
- Rotating disc speed: : 433 rpm
- Inlet Temperature: : 60°C
- Air vol.: : 4.7-5.3

| Time in mins. | Outlet air temp.°C | Spray Rate g/min |
|---|---|---|
| 0 | 50 | 11 |
| 8 | 54 | 8.5 |
| 19 | 59 | 11 |
| 23 | 52 | 26 |
| 34 | 52 | 36 |
| 55 | 48 | 40 |
| 68 | 46 | 45 |
| 84 | 45 | 49 |
| 102 | 44 | 52 |
| 114 | 43 | 54 |
| 270 | 41 | 56 |

Spraying was complete after 295 mins. The beadlets were allowed to dry for 5 mins.
- Theoretical drug load: = 60% w/w
- Actual drug load: = 59.1% w/w
- Fill weight per size 0 capsule: = 498mg

### Example 5

### Preparation of Pseudoephedrine HCl Beadlets Using 600-500µm(30-35#) Non-Pareil Seeds

1.5kg of non-pareil seeds were placed in the bowl of the rotoprocessor. 6.583kg of pseudoephedrine HCl solution (38% w/w in filtered tap water) with no binder present was sprayed onto the seeds. Spray rate was increased as processing progressed.
- Rotating disc speed: : 433 rpm
- Inlet Temperature: : 60°C
- Air vol.: : 4.5-6.0

| Time in mins | Outlet air temp.°C | Spray Rate g/min |
|---|---|---|
| 0 | 47 | 9 |
| 10 | 52 | 13 |
| 20 | 51 | 20 |
| 22 | 50 | 25 |
| 30 | 47 | 27 |
| 35 | 46 | 28 |
| 40 | 47 | 31 |
| 50 | 46 | 32 |
| 55 | 46 | 36 |
| 80 | 44 | 38 |
| 90 | 42 | 43 |
| 110 | 39 | 44 |
| 130 | 40 | 46 |
| 140 | 39 | 49 |
| 160 | 37 | 52 |

Spraying was complete after 175 mins.
- Theoretical drug load: = 62.5% w/w
- Actual drug load: = 60.7% w/w
- Fill weight per size 0 capsule: = 504mg

### Example 6

### Preparation of Pseudoephedrine HCl Sustained Release Beadlets

Pseudoephedrine HCl beadlets prepared by the method of Example 5 were coated with sustained release polymers to provide a sustained release of pseudoephedrine HCl after oral dosing.

| Raw Material | % w/w |
|---|---|
| Aquacoat | 40.70 |
| Citroflex 2 | 2.80 |
| Quinoline Yellow | 0.12 |
| Deionised Water | 56.38 |
| Total Suspenion | 100.00 |

### Method

A suspension of Aquacoat plasticized with Citroflex was sprayed onto the pseudoephedrine beadlets (62.5% drug) using a bottom spray technique. The coat was sprayed onto the beadlets to give 4% slow coat plus 30% top coat (% w/w of the total beads). Slow coat was sprayed onto the beadlets slowly to seal water soluble drug into the beadlets and prevent dissolution into the coat.

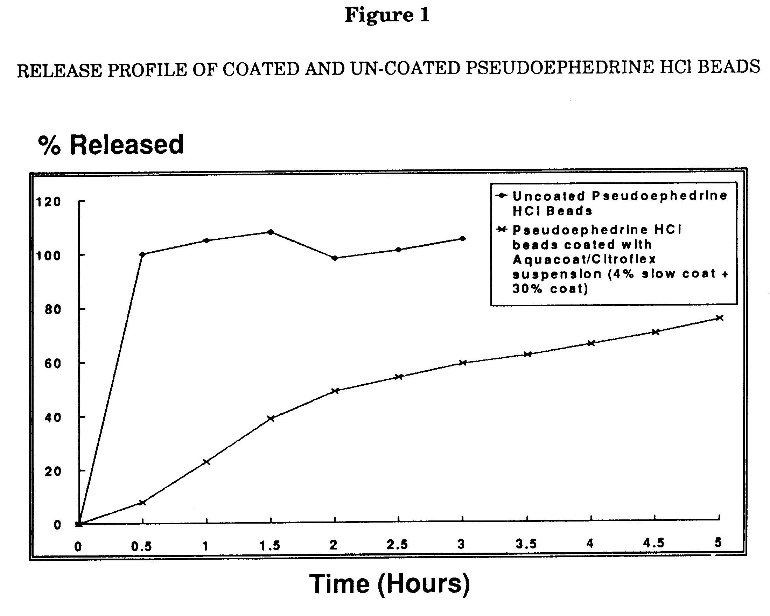

Figure 1 shows the dissolution profile of the coated beads in comparison to the beadlets of Example 5. A BP dissolution apparatus with paddles and 1 litre of distilled water per dissolution cell was used.

## Claims

1. A process for the preparation of drug substances in beadlet form comprising: forming a fluidised bed of particles of inert core material within the processing chamber of a centrifugal, fluidised bed coater-granulator apparatus by passing a gaseous current between the rotating disc and the inner wall of the processing chamber; and forming beadlets by layering a drug onto the particles of the core material by spray-coating with a solution of the drug; characterised in that during beadlet formation, the spray-coated particles are (i) transferred to a drying zone surrounding the processing chamber, (ii) carried through the drying zone by a gaseous current, and (iii) returned to the processing chamber; the recirculation process being continued until beadlets having the desired drug load are formed.

2. A process as claimed in Claim 1 wherein spray-coated particles are transferred to the drying zone surrounding the processing chamber through an annular ring gap in the wall of the processing chamber.

3. A process as claimed in claim 1 or claim 2 in which the centrifugal, fluidised bed coater-granulator apparatus is provided with a tangentially aligned, adjustable spray-rate, spray-coating system.

4. A process as claimed in any one of claims 1 to 3 in which the drug substance is selected from one or more of ibuprofen, pseudoephedrine, phenylpropanolamine, chlorpheniramine, dextromethorphan and diphenhydramine or pharmaceutically acceptable salts thereof.

5. A process as claimed in any one of claims 1 to 4 wherein drug is spray-coated onto core particles from a solution comprising a lower alkyl aliphatic alcohol, acetone or water, or mixtures thereof.

6. A process as claimed in any one of claims 1 to 5 wherein at least two drug substances are layered sequentially onto core particles.

7. A process as claimed in any one of claims 1 to 5 wherein at least two drug substances are deposited onto core particles as a single layer.

8. A process as claimed in any one of claims 1 to 7 wherein the concentration of drug in the spray-coating solution is in the range 20 to 60% w/w.

9. A process as claimed in any one of claims 1 to 8 wherein the particles of inert core material are non-pareil seeds with mesh size in the range 850-410µm(20-40#).

10. A process as claimed in any one of claims 1 to 9 in which the spray-coating solution further comprises a binder material.

11. A process as claimed in claim 10 wherein the binder material is selected from gelatin, starch, polyvinylpyrrolidone, hydroxypropylcellulose, hydroxymethylpropylcellulose and carboxymethylcellulose and mixtures thereof.

12. A process as claimed in claim 10 or 11 wherein the spray-coating solution comprises less than 1.0% of binder material.

13. A process as claimed in any one of claims 1 to 9 wherein binder material is not present in the spray-coating solution.

14. A process as claimed in any one of claims 1 to 13 comprising a further process step in which beadlets are film-coated. 15. A process as claimed in claim 14 in which the film-coating

15. comprises an acrylate or cellulose based polymer.

16. A process as claimed in any one of claims 1 to 15 providing a drug beadlet with a fill weight per size 0 capsule in excess of 450 mg.

17. A drug beadlet obtainable by a process as defined in any one of claims 1 to 16.

18. A drug beadlet as claimed in claim 17 which comprises ibuprofen.

## Patentansprüche

1. Verfahren zur Herstellung von Arzneimittelsubstanzen in Form von Kügelchen, umfassend: Bilden eines Fliessbettes aus Partikeln aus inertem Kernmaterial innerhalb der Prozesskammer eines Rotor-Wirbelschicht-Überzugsgranulators durch Hindurchleiten eines Gasstroms zwischen dem Rotor und der Innenwand der Prozesskammer; und Bilden von Kügelchen durch Beschichten der Partikel des Kernmaterials durch Sprühüberziehen mit einer Lösung des Arzneistoffs; dadurch **gekennzeichnet,** dass während der Bildung der Kügelchen die sprühüberzogenen Partikel (i) in eine die Prozesskammer umgebende Trocknungszone überführt, (ii) mittels eines Gasstroms durch die Trocknungszone getragen und (iii) zur Prozesskammer zurückgeführt werden; wobei das Rücknahmeverfahren fortgesetzt wird, bis Kügelchen mit der gewünschten Arzneistoffmenge gebildet sind.

2. Verfahren gemäss Anspruch 1, in dem sprühüberzogene Partikel in die Trocknungszone, die die Prozesskammer umgibt, durch einen Ringspalt in der Wand der Prozesskammer überführt werden.

3. Verfahren gemäss Anspruch 1 oder Anspruch 2, in dem der Rotor-Wirbelschicht-Überzugsgranulator mit einem tangential ausgerichteten Sprühüberzugssystem mit einstellbarer Sprührate versehen ist.

4. Verfahren gemäss einem der Ansprüche 1 bis 3, in dem die Arzneimittelsubstanz ausgewählt ist aus einem oder mehreren Vertretern aus Ibuprofen, Pseudoephedrin, Phenylpropanolamin, Chlorpheniramin, Dextromethorphan und Diphenhydramin oder pharmazeutisch annehmbaren Salzen davon.

5. Verfahren gemäss einem der Ansprüche 1 bis 4, in dem die Kernpartikel mit dem Arzneistoff aus einer Lösung, umfassend einen aliphatischen Niederalkylalkohol, Aceton oder Wasser oder Mischungen davon, sprühüberzogen werden.

6. Verfahren gemäss einem der Ansprüche 1 bis 5, in dem die Kernpartikel mit wenigstens zwei Arzneimittelsubstanzen hintereinander beschichtet werden.

7. Verfahren gemäss einem der Ansprüche 1 bis 5, in dem wenigstens zwei Arzneimittelsubstanzen als Einzelschicht auf die Kernpartikel aufgetragen werden.

8. Verfahren gemäss einem der Ansprüche 1 bis 7, in dem die Arzneistoffkonzentration in der Sprühüberzugslösung im Bereich von 20 bis 60 % G/G ist.

9. Verfahren gemäss einem der Ansprüche 1 bis 8, in dem die Partikel des inerten Kernmaterials Non-pareil-Kristallkeime mit einer Maschengrösse im Bereich von 850 bis 410 µm (20 bis 40 #) sind.

10. Verfahren gemäss einem der Ansprüche 1 bis 9, in dem die Sprühüberzugslösung zusätzlich ein Bindemittel umfasst.

11. Verfahren gemäss Anspruch 10, in dem das Bindemittel ausgewählt wird aus Gelatine, Stärke, Polyvinylpyrrolidon, Hydroxypropylcellulose, Hydroxymethylpropylcellulose und Carboxymethylcellulose und Mischungen davon.

12. Verfahren gemäss Anspruch 10 oder 11, in dem die Sprühüberzugslösung weniger als 1,0 % Bindemittel umfasst.

13. Verfahren gemäss einem der Ansprüche 1 bis 9, in dem kein Bindemittel in der Sprühüberzugslösung vorhanden ist.

14. Verfahren gemäss einem der Ansprüche 1 bis 13, umfassend einen weiteren Verfahrensschritt, in dem Kügelchen filmüberzogen werden.

15. Verfahren gemäss Anspruch 14, in dem der Filmüberzug ein Polymer auf Acrylat- oder Cellulosebasis umfasst.

16. Verfahren gemäss einem der Ansprüche 1 bis 15, das ein Arzneistoffkügelchen mit einem Füllgewicht je Kapsel von Grösse 0 von mehr als 450 mg liefert.

17. Arzneistoffkügelchen, erhältlich durch ein Verfahren gemäss einem der Ansprüche 1 bis 16.

18. Arzneistoffkügelchen gemäss Anspruch 17, das Ibuprofen umfasst.

## Revendications

1. Procédé de préparation de principes actifs sous forme de petites billes comprenant : la formation d'un lit fluidisé de noyaux constitués d'un matériau inerte, à l'intérieur de la chambre de mélange d'un appareil granulateur-enrobeur centrifuge à lit fluidisé, par passage d'un courant gazeux entre le disque rotatif et la paroi intérieure de la chambre de mélange ; et la formation de petites billes en déposant un principe actif sur les particules de matériau constituant les noyaux, par enrobage par pulvérisation d'une solution du principe actif;
caractérisé en ce qu'au cours de la formation des petites billes, les particules enrobées par pulvérisation sont : (i) transférées vers une zone de séchage entourant la chambre de mélange, (ii) véhiculées par un courant gazeux, à travers la zone de séchage, et (iii) renvoyées dans la chambre de contact ; le processus de recirculation étant poursuivi jusqu'à ce que les petites billes formées aient la charge désirée en principe actif.

2. Procédé selon la revendication 1, dans lequel les particules enrobées par pulvérisation sont transférées dans la zone de séchage entourant la chambre de mélange à travers un espace annulaire circulaire ménagé dans la paroi de la chambre de mélange.

3. Procédé selon la revendication 1, ou la revendication 2, dans lequel l'appareil granulateur-enrobeur centrifuge à lit fluidisé est muni d'un système d'enrobage par pulvérisation, à débit de pulvérisation réglable, aligné tangentiellement.

4. Procédé selon l'une quelconque des revendications 1 à 3, dans lequel le principe actif est choisi parmi un ou plusieurs membres du groupe comprenant l'ibuprofène, la pseudoéphédrine, la phénylpropanolamine, la chlorphéniramine, le dextrométhorphan, et la diphenhydramine, ou les sels pharmaceutiquement acceptables de ceux-ci.

5. Procédé selon l'une quelconque des revendications 1 à 4, dans lequel principe actif est déposé par pulvérisation sur les particules constituants les noyaux, d'une solution contenant un alcool aliphatique alkyle inférieur, de l'acétone, ou de l'eau, ou des mélanges de ceux-ci.

6. Procédé selon l'une quelconque des revendications 1 à 5, dans lequel au moins deux principes actifs sont déposés séquentiellement sur les particules constituant les noyaux.

7. Procédé selon l'une quelconque des revendications 1 à 5, dans lequel au moins deux principes actifs sont déposés sur les particules constituant les noyaux sous la forme d'une couche unique.

8. Procédé selon l'une quelconque des revendications 1 à 7, dans lequel la concentration du principe actif dans la solution d'enrobage par pulvérisation est comprise entre 20 et 60 % p/p.

9. Procédé selon l'une quelconque des revendications 1 à 8, dans lequel les particules constituant les noyaux de matériau inerte sont des grains non homogènes, d'une taille comprise entre 850 µm et 410 µm (tamis n° 20-40).

10. Procédé selon l'une quelconque des revendications 1 à 9, dans lequel la solution d'enrobage par pulvérisation contient aussi un matériau liant.

11. Procédé selon la revendication 10, dans lequel le matériau liant est choisi parmi la gélatine, l'amidon, la polyvinylpyrrolidone, l'hydroxypropylcellulose, l'hydroxyméthylpropylcellulose, et la carboxyméthylcellulose, et les mélanges de ceux-ci.

12. Procédé selon la revendication 10 ou la revendication 11, dans lequel la solution d'enrobage par pulvérisation contient moins de 1,0 % de matériau liant.

13. Procédé selon l'une quelconque des revendications 1 à 9, dans lequel le matériau liant n'est pas présent dans la solution d'enrobage par pulvérisation.

14. Procédé selon l'une quelconque des revendications 1 à 13, comprenant une étape additionnelle d'opération au cours de laquelle les petites billes sont pelliculées.

15. Procédé selon la revendication 14, dans lequel le revêtement pelliculaire contient un polymére à base d'acrylate ou de cellulose.

16. Procède selon l'une quelconque des revendications 1 à 15, donnant une petite bille de médicament, avec un poids de remplissage par capsule de calibre 0, supérieur à 450 mg.

17. Petite bille de médicament pouvant être obtenue par un procédé selon l'une quelconque des revendications 1 à 16.

18. Petite bille de médicament selon la revendication 17, qui contient de l'ibuprofène.
